(19) Europäisches Patentamt / European Patent Office / Office européen des brevets

(11) **EP 4 653 416 A1**

(12) **EUROPEAN PATENT APPLICATION**
published in accordance with Art. 153(4) EPC

(43) Date of publication:
**26.11.2025 Bulletin 2025/48**

(21) Application number: **24924254.6**

(22) Date of filing: **20.12.2024**

(51) International Patent Classification (IPC):
$C07C\ 51/02^{(2006.01)}$  $C07C\ 51/41^{(2006.01)}$
$C07C\ 51/09^{(2006.01)}$  $C07C\ 51/47^{(2006.01)}$
$C07C\ 63/26^{(2006.01)}$  $C07C\ 63/28^{(2006.01)}$
$C08J\ 11/24^{(2006.01)}$

(52) Cooperative Patent Classification (CPC):
**C07C 51/02; C07C 51/09; C07C 51/41;
C07C 51/47; C07C 63/26; C07C 63/28; C08J 11/24;
Y02W 30/62**

(86) International application number:
**PCT/KR2024/020791**

(87) International publication number:
**WO 2025/206524 (02.10.2025 Gazette 2025/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.04.2024 KR 20240057836**

(71) Applicant: **Terracle Co., Ltd.**
**Busan 48059 (KR)**

(72) Inventors:
• **LEE, Seong Eon**
 **Incheon 22640 (KR)**
• **LEE, Tea Geon**
 **Incheon 22775 (KR)**

(74) Representative: **Isarpatent**
**Patent- und Rechtsanwälte
Barth Hassa Peckmann & Partner mbB
Friedrichstraße 31
80801 München (DE)**

(54) **METHOD USING POLYESTER DEPOLYMERIZATION FOR PRODUCING HIGHLY PURE TEREPHTHALIC ACID AND HIGHLY PURE TEREPHTHALIC ACID OBTAINED THEREBY**

(57)    The present disclosure provides a method for producing high-purity regenerated terephthalic acid using polyester depolymerization and high-purity regenerated terephthalic acid produced thereby. An object to be achieved by the present disclosure is to provide a method for producing terephthalic acid capable of obtaining high-purity terephthalic acid from waste polyester, reducing the amount of solvent used for hydrolysis, solvent recovery energy, and manufacturing cost, and increasing the stability of the solvent.

【 FIG. 1 】

(X AXIS: TIMES, Y AXIS: CONVERSION RATE)

**Description**

**[TECHNICAL FIELD]**

The present disclosure relates to a method for obtaining high-purity terephthalic acid from waste polyester. In addition, the present disclosure relates to the regeneration of high-purity terephthalic acid with improved conversion rate into terephthalic acid by increasing the purification efficiency using an alkylated aromatic compound having a stable structure even under basic conditions and controlling the polarity of the solvent so that a swelling process of waste plastic may easily occur.

**[BACKGROUND ART]**

Polyester is a polymer which is the most widely purchased and widely utilized worldwide, and is mass-produced in excess of 75 million tons annually. The polyester may be produced at a relatively low cost and is used for various applications, including clothing, carpets, and films due to its physical, chemical, and thermal properties. Among polyesters, polyethylene terephthalate (PET) is one of the polyesters, that are widely used in various methods, including disposable applications such as beverage containers. Among the polyesters, particularly, PET has become commercially successful, and efforts have been made to recover materials from post-consumption, post-industrial use, scrap, and other sources, and to reuse these materials as an alternative of basic disposal methods such as landfill. As the annual global volume of plastic waste made of these polyesters is difficult to handle, interest in recycling waste polyester or a regeneration process using waste polyester is increasing. Among waste polyesters, in the case of beverage containers using PET, colorless and transparent containers may be physically recycled, but colored and composite materials such as automobile interior materials or fibers are difficult to be recycled, and have been incinerated or landfilled. Accordingly, chemically recycling technologies have been provided as an alternative of basic disposal methods such as incineration or landfill by recovering waste polyester.

One of methods for chemically recycling waste polyester is a depolymerization technology, which is a technology that converts polyester into monomers and re-polymerizes the monomers generated by depolymerization to be reproduced into new products. These depolymerization technologies may be divided into glycolysis, methanolysis, and hydrolysis.

The glycolysis is a method of monomerizing polyester into bis(2-hydroxyethyl) terephthalate (BHET) through an ester exchange reaction using glycols such as ethylene glycol (EG) or propylene glycol (PG) and a catalyst. The glycolysis is the simplest, oldest, and easiest process to attempt, and is a technology that has been developed by many companies. However, the glycolysis is very slow when a catalyst such as metal salt, zeolite, or ionic catalyst is not used, and complete depolymerization of PET into BHET may not be achieved. Since the final product containing a significant amount of other oligomers in addition to the BHET monomers is obtained through glycolysis, it is difficult to recover the BHET monomers when the BHET monomers are a desired product. Since the glycolysis is carried out at a high temperature of 150°C or higher, there are problems occurring in terms of energy efficiency and reduction of carbon emissions. In addition, a pretreatment process to remove heavy metals and moisture in waste is required prior to the glycolysis reaction, and since a significant amount of activated carbon is used during the purification, there is a disadvantage of being not economically feasible. Furthermore, although BHET produced by glycolysis is suitable for PET synthesis, there are problems of having poor economic feasibility and poor versatility because the BHET requires an additional hydrolysis reaction to manufacture high value-added polyesters such as polybutylene terephthalate (PBT).

The methanolysis is a method of depolymerizing polyester into dimethyl terephthalate (DMT) and ethylene glycol through an ester exchange reaction using methanol and a catalyst. The methanolysis has the same reaction mechanism as glycolysis, but has the advantage of being able to react even at low temperatures below 100°C, and thus a lot of commercial development has been conducted. The methanolysis has the advantage of being able to produce various polyesters, and the produced monomer, DMT, is also characterized by high versatility. However, the methanolysis also requires a pretreatment process of removing heavy metals and moisture in the waste before the reaction, and has a disadvantage of being not economically feasible and not good for the environment because sublimated purification is performed at a high temperature of 200°C or higher during purification. In addition, currently, polyester production facilities around the world have polymerization facilities using a terephthalic acid (TPA) method, and thus have a problem with usability.

The hydrolysis is a method of depolymerizing polyester into terephthalic acid and ethylene glycol through hydrolysis using water or a solvent and a catalyst. The hydrolysis is largely divided into acid hydrolysis, neutral hydrolysis, and alkaline hydrolysis. The acid hydrolysis is a reaction in which water and sulfuric acid are used, heated to a temperature of 100°C to directly obtain terephthalic acid. This reaction has the advantage of being able to obtain terephthalic acid immediately and not using an organic solvent, but there is a risk of using at least 85% sulfuric acid, and the terephthalic acid produced immediately needs to be alkalized again to be purified, so that it is not economical. The neutral hydrolysis is a method of using high-temperature steam, and terephthalic acid is obtained at a high temperature of 200 to 300°C or by irradiating microwaves. However, this method requires special equipment and consumes a lot of energy. In addition, the process of alkalization again for purification is the same as that of acid hydrolysis. The alkaline hydrolysis is a process in which an intermediate terephthalate salt is formed using an alkaline catalyst and water or a solvent, and then a purification process is performed to form the final terephthalic acid through acid treatment. The alkaline hydrolysis has the advantage of having relatively simple reaction conditions and facilities and mild conditions, but is poorly economical and environmentally friendly because a large amount of water and solvent are used in the purification process. In addition, the alkaline hydrolysis has the disadvantage of forming a high unit price by requiring a large amount of activated carbon to remove non-specific impurities such as dyes and an expensive solvent for a high conversion rate. In other words, the hydrolysis has a problem of requiring a plurality of steps to recover terephthalic acid monomers.

Therefore, there is a continuing need for a method for regenerating waste polyester through a low-cost solvent composition that is easy to recover terephthalic acid, may recover terephthalic acid with high purity, and is highly stable even when used repeatedly.

[Prior Arts]

[Patent Document]

Korean Patent No. 10-2462599

[DISCLOSURE]

[TECHNICAL PROBLEM]

An object to be achieved by the present disclosure is to provide a method for producing terephthalic acid capable of obtaining high-purity terephthalic acid from waste polyester, reducing the amount of solvent used for hydrolysis, solvent recovery energy, and manufacturing cost, and increasing the stability of the solvent.

[TECHNICAL SOLUTION]

According to an aspect of the present disclosure, a method for producing high-purity regenerated terephthalic acid using polyester depolymerization according to an embodiment of the present disclosure may include preparing a solvent by mixing an alkaline catalyst with a basic hydrolysis solvent consisting of an alkylated aromatic compound and a polarity adjusting compound, obtaining a polyester depolymerization product by treating waste polyester with the solvent, preparing a sludge cake aqueous solution containing a terephthalate metal salt by filtering the polyester depolymerization product to obtain a sludge cake and adding purified water to the filtered sludge cake, obtaining an aqueous solution containing a terephthalate metal salt by treating the sludge cake aqueous solution with an organic solvent, purifying the terephthalate metal salt aqueous solution by treating the aqueous solution with an adsorbent, and precipitating solid terephthalic acid by treating the purified terephthalate metal salt aqueous solution with an acidic solution.

[ADVANTAGEOUS EFFECTS]

According to the method for producing terephthalic acid using polyester depolymerization according to the present disclosure, the conversion rate to terephthalic acid is very high, and the regenerated terephthalic acid contains almost no impurities, thereby providing high-purity terephthalic acid. In addition, it is possible to minimize the amount of solvent used in the conversion to terephthalic acid and the solvent recovery energy, and to be used repeatedly due to high stability of the solvent.

[DESCRIPTION OF DRAWINGS]

FIG. 1 is a graph showing the conversion rate of toluene used in Example 17 of the present disclosure.
FIG. 2 is a graph showing the conversion rate of toluene used in Example 18 of the present disclosure.
FIG. 3 is a graph showing the conversion rate of toluene used in Example 19 of the present disclosure.
FIG. 4 is a graph showing the conversion rate of anisole used in Comparative Example 3 of the present disclosure.
FIG. 5 is a graph showing the conversion rate of MIBK used in Comparative Example 4 of the present disclosure.

**[BEST MODE]**

Hereinafter, various exemplary embodiments of the present disclosure will be described in detail with reference to the accompanying drawings. The present disclosure is not limited to specific exemplary embodiments, and it should be understood to include various modifications, equivalents, and/or alternatives to the exemplary embodiments of the present disclosure. In connection with the description of the drawings, similar reference numerals may be used for similar components.

In this specification, expressions such as "have," "may have," "include," or "may include" refer to the presence of the corresponding feature (e.g., numerical value, function, operation, or component such as part), and does not exclude the presence of additional features.

In the present disclosure, the expression such as "A or B", "at least one of A and/or B", or "one or more of A and/or B" may include all possible combinations of items listed together. For example, "A or B", "at least one of A and B", or "at least one of A or B" may refer to all cases of (1) including at least one A, (2) including at least one B, or (3) including both at least one A and at least one B.

The expression of "configured to" used herein may be changed and used to, for example, "suitable for", "having the capacity to", "designed to", "adapted to", "made to" or "capable of", depending on the situation. The term of "configured to" may not necessarily mean "specially designed to".

The terms used herein are used to illustrate only specific exemplary embodiments, and may not be intended to limit the scope of other exemplary embodiments. A singular expression may include a plural expression unless the context clearly indicates otherwise. The terms used herein, including technical or scientific terms, may have the same meaning as generally understood by those of ordinary skill in the art described in the present disclosure. The terms defined in a general dictionary among the terms used herein may be interpreted in the same or similar meaning as or to the meaning on the context of the related art, and will not be interpreted as an ideal or excessively formal meaning unless otherwise defined in the present disclosure. In some cases, even the terms defined in the present disclosure may not be interpreted to exclude the exemplary embodiments of the present disclosure.

The exemplary embodiments disclosed in the present disclosure are presented for explanation and understanding of the disclosed technical contents, and do not limit the scope of the present disclosure. Therefore, the scope of the present disclosure should be interpreted as including all changes or various other exemplary embodiments based on the technical idea of the present disclosure.

Hereinafter, a preferred exemplary embodiment of the present disclosure will be described in detail. Terms and words used in the present specification and claims should not be interpreted as being limited to typical or dictionary meanings, but should be interpreted as having meanings and concepts which comply with the technical spirit of the present disclosure, based on the principle that an inventor may appropriately define the concept of the term to describe his/her own invention in the best manner.

Therefore, the configurations of the exemplary embodiments described in the present specification are merely the most preferred exemplary embodiment of the present disclosure and are not intended to represent all of the technical ideas of the present disclosure, and thus, it should be understood that there are various equivalents and modifications capable of replacing the configurations at the time of this application.

Throughout the specification, when a part "includes" a component, unless otherwise specifically stated, it is meant to further include other components rather than excluding other components.

Hereinafter, the present disclosure will be described in detail.

A method for producing high-purity regenerated terephthalic acid using polyester depolymerization according to an embodiment of the present disclosure may include preparing a solvent by mixing an alkaline catalyst with a basic hydrolysis solvent consisting of an alkylated aromatic compound and a polarity adjusting compound, obtaining a polyester depolymerization product by treating waste polyester with the solvent, preparing a sludge cake aqueous solution containing a terephthalate metal salt by filtering the polyester depolymerization product to obtain a sludge cake and adding purified water to the filtered sludge cake, obtaining an aqueous solution containing a terephthalate metal salt by treating the sludge cake aqueous solution with an organic solvent, purifying the terephthalate metal salt aqueous solution by treating the aqueous solution with an adsorbent, and precipitating solid terephthalic acid by treating the purified terephthalate metal salt aqueous solution with an acidic solution.

The preparing of the solvent by mixing the alkaline catalyst with the basic hydrolysis solvent consisting of the alkylated aromatic compound and the polarity adjusting compound is for preparing a solvent having a stable structure capable of sufficiently swelling the polyester structure and increasing the conversion rate into terephthalic acid.

The polyester is a polymer having an ester (RO-C(=O)-R') chemical functional group in a main chain, which is also called polyester.

The polyester may be a polymer formed by condensation polymerization of dicarboxylic acid and dialcohol. The dicarboxylic acid may be any one selected from the group consisting of terephthalic acid, naphthalene dicarboxylic acid, diphenyl dicarboxylic acid, diphenyl ether dicarboxylic acid, diphenyl sulfone dicarboxylic acid, diphenoxyethane dicarboxylic acid, succinic acid, adipic acid, sebacic acid, azelaic acid, decanedicarboxylic acid, cyclohexanedicarboxylic acid, trimellitic acid, and pyromellitic acid. Preferably, the dicarboxylic acid may be terephthalic acid.

The dialcohol may be any one selected from the group consisting of ethylene glycol, trimethylene glycol, 1,2-propanediol, tetramethylene glycol, neopentyl glycol, hexamethylene glycol, decanmethylene glycol, dodecamethylene glycol, 1,4-cyclohexanedimethanol, diethylene glycol, triethylene glycol, tetraethylene glycol, polyethylene glycol, dipropylene glycol, tripropylene glycol, tetrapropylene glycol, polypropylene glycol, di(tetramethylene) glycol, tri(tetramethylene) glycol, polytetramethylene glycol, pentaerythritol, and 2,2-bis(4-βhydroxyethoxyphenyl)propane. Preferably, the dialcohol may be ethylene glycol.

The polyester may be at least one selected from the group consisting of polyethylene terephthalate (PET), polypropylene terephthalate (PPT), polyglycolide or polyglycolic acid (PGA), polylactic acid (PLA), polycaprolactone (PCL), polyhydroxyalkanoate (PHA), polyhydroxybutyrate (PHB), polyethylene adipate (PEA), polybutylene succinate (PBS), poly(3-hydroxybutyrate-co-3-hydroxyvalerate) (PHBV), polybutylene terephthalate (PBT), polytrimethylene terephthalate (PTT), polyethylene naphthalate (PEN), and Vectran. Preferably, the polyester may be polyethylene terephthalate (PET).

In the preparing of the solvent by mixing the alkaline catalyst with the basic hydrolysis solvent consisting of the alkylated aromatic compound and the polarity adjusting compound, the solvent is used for the basic hydrolysis of polyester and may sufficiently dissolve the alkaline catalyst and may be used repeatedly. The alkylated aromatic compound has a structure highly stable against the basic hydrolysis reaction of polyester and may produce high-purity terephthalic acid. The polarity adjusting compound may increase the solubility of the catalyst and easily affect the destruction of the ester functional group. The alkaline catalyst mixed in the preparing of the solvent forms a form of a terephthalate metal salt and is dissolved in purified water to be added after filtration to create an alkaline environment, so as to help completely decompose monoesters, which are by-products of the hydrolysis reaction to lead to complete hydrolysis.

In the preparing of the solvent by mixing the alkaline catalyst with the basic hydrolysis solvent consisting of the alkylated aromatic compound and the polarity adjusting compound, the alkylated aromatic compound may be at least one compound selected from the group consisting of toluene, xylene, trimethylbenzene, ethylbenzene, diethylbenzene, propylbenzene, dipropylbenzene, and butylbenzene. Preferably, the alkylated aromatic compound may be toluene. The alkylated aromatic compound has a stable structure even under basic conditions, does not generate contaminants even by a chain reaction, and enables reuse of the solvent.

In the preparing of the solvent by mixing the alkaline catalyst with the basic hydrolysis solvent consisting of the alkylated aromatic compound and the polarity adjusting compound, the polarity adjusting compound may be at least one compound selected from the group consisting of methanol, ethanol, propanol, butanol, pentanol, hexanol, heptanol, octanol, nonanol, decanol, undecanol, dodecanol, ethylene glycol, propylene glycol and butylene glycol. Preferably, the polarity adjusting compound may be ethanol. The polarity adjusting compound may serve to increase the solubility of the alkaline catalyst and adjust the polarity of the solvent to facilitate swelling of the polyester.

In the preparing of the solvent by mixing the alkaline catalyst with the basic hydrolysis solvent consisting of the alkylated aromatic compound and the polarity adjusting compound, the alkaline catalyst may be at least one selected from the group consisting of potassium hydroxide, sodium hydroxide, lithium hydroxide, potassium oxide, sodium oxide, and lithium oxide. The alkaline catalyst prevents a portion of the prepared terephthalic acid from acting as an acid catalyst and forms a form of a terephthalate-metal salt to be easily dissolved in water.

In the preparing of the solvent by mixing the alkaline catalyst with the basic hydrolysis solvent consisting of the alkylated aromatic compound and the polarity adjusting compound, the weight ratio of the alkylated aromatic compound and the polarity adjusting compound may be 1:0.1 to 10. Preferably, the weight ratio may be 1:0.5 to 5. Most preferably, the weight ratio may be 1:0.6 to 3. The solvent to be added for the basic hydrolysis of polyester needs to be able to sufficiently dissolve the basic catalyst and is affected by the polarity of the solvent. Therefore, if the weight of the polarity adjusting compound is less than the numerical range, the conversion rate to terephthalic acid is low and the reaction needs to be performed under high temperature conditions to increase the conversion rate. If the weight exceeds the numerical range, the polarity increases to be difficult to swell plastics.

In the preparing of the solvent by mixing the alkaline catalyst with the basic hydrolysis solvent consisting of the alkylated aromatic compound and the polarity adjusting compound, the weight ratio of the basic hydrolysis solvent and the alkaline catalyst may be 1:0.01 to 0.5. Preferably, the weight ratio may be 1:0.05 to 0.3. If the weight of the alkaline catalyst is less than the numerical range, the metal content derived from the catalyst may be low, which may lower the yield of terephthalic acid, and if the weight of the alkaline catalyst exceeds the numerical range, the polarity adjusting compound may be excessively mixed to dissolve the catalyst, which may lower the conversion rate into terephthalic acid.

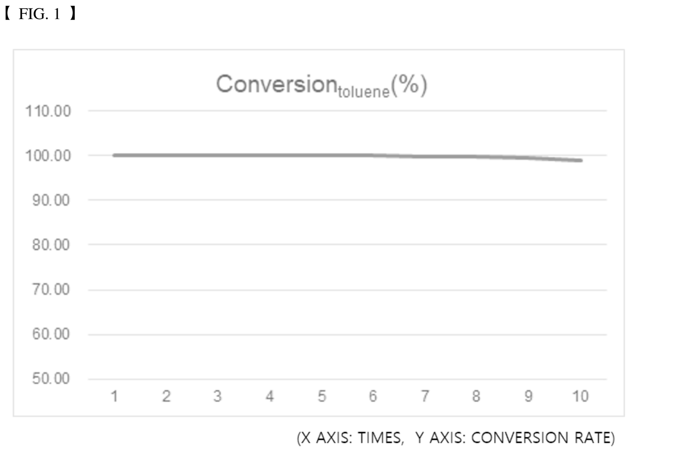

In the preparing of the solvent by mixing the alkaline catalyst with the basic hydrolysis solvent consisting of the alkylated aromatic compound and the polarity adjusting compound, the basic hydrolysis solvent consisting of the alkylated aromatic compound and the polarity adjusting compound is characterized in that the conversion rate of the alkylated aromatic compound at the 10th time is 90% or higher as a solvent reuse rate (see FIGS. 1 to 3).

The obtaining of the polyester depolymerization product by treating the waste polyester with the solvent is for eluting a terephthalate metal salt, and may produce environmentally friendly and high-purity terephthalic acid by treating the solvent. The step may involve a saponification reaction along with a hydrolysis reaction via water generated by the basic hydrolysis solvent and the alkaline catalyst.

In the obtaining of the polyester depolymerization product by treating the waste polyester with the solvent, an ester functional group of the polyester is destroyed by the treating polyester and waste polyester such as bulk waste that has not undergone a separate pulverization step may also be depolymerized. In addition, in the process of performing basic hydrolysis by treating the waste polyester with the solvent, conditions such as a temperature and a pressure may be applied without limitation to the conditions applied in the previous hydrolysis method.

In the obtaining of the polyester depolymerization product by treating the waste polyester with the solvent, the weight ratio of the waste polyester and the solvent may be 1:1 to 50. Preferably, the mixing weight ratio may be 1:5 to 25. Most preferably, the weight ratio of the waste polyester and the solvent may be 1:5 to 20. If the weight of the solvent is less than the numerical range, the viscosity of the waste polyester treated with the solvent increases, so that the depolymerization may not occur smoothly, and if the weight of the solvent exceeds the numerical range, the solvent is added too much, which is not economical.

In the obtaining of the polyester depolymerization product by treating the waste polyester with the solvent, the solvent may be added so that the pH of the entire reaction is 7.1 or higher.

The preparing of the sludge cake aqueous solution containing the terephthalate metal salt by filtering the polyester depolymerization product to obtain the sludge cake and adding the purified water to the filtered sludge cake is to remove the solvent and dissolved impurities from the polyester depolymerization product. Since the filtered sludge cake contains the obtained terephthalate metal salt and non-reactive impurities, the purified water is added thereto to obtain an aqueous solution containing the first purified terephthalate metal salt.

The terephthalate metal salt is bonded with an alkali metal derived from a catalyst, and may be any one selected from the group consisting of dipotassium terephthalate ($K_2$-TPA), disodium terephthalate ($Na_2$-TPA), and dilithium terephthalate ($Li_2$-TPA). Preferably, the terephthalate metal salt may be disodium terephthalate ($Na_2$-TPA).

In the preparing of the sludge cake aqueous solution containing the terephthalate metal salt by filtering the polyester depolymerization product to obtain the sludge cake and adding the purified water to the filtered sludge cake, the weight ratio of the filtered sludge cake and the purified water may be 1:2 to 20. Preferably, the weight ratio may be 1:4 to 15. Most preferably, the weight ratio may be 1:4 to 10. If the amount of purified water added is less than the numerical range, the filtered sludge cake reaches a supersaturated state, and if the amount of purified water exceeds the numerical range, the filtered sludge cake has a negative impact on the environment and poor economic feasibility.

The obtaining of the aqueous solution containing the terephthalate metal salt by treating the sludge cake aqueous solution with the organic solvent is to perform phase separation by adding an organic solvent for purification.

In the obtaining of the aqueous solution containing the terephthalate metal salt by treating the sludge cake aqueous solution with the organic solvent, the organic solvent may be at least one selected from the group consisting of pentane, hexane, heptane, octane, nonane, decane, and undecane. The organic solvent may be used as a hydrophobic solvent alone or may be used by mixing different components of hydrophobic solvents. In some cases, the organic solvent may be used as a water-soluble solvent alone or mixed with a hydrophobic solvent. The organic solvent may be used for the purpose of removing impurities or reaction solvents present in a trace amount in an aqueous layer after phase separation.

In the obtaining of the aqueous solution containing the terephthalate metal salt by treating the sludge cake aqueous solution with the organic solvent, the weight ratio of the sludge cake aqueous solution and the organic solvent may be 1:1 to 20. Preferably, the weight ratio may be 1:2 to 15. Most preferably, the weight ratio may be 1:2 to 10. If the organic solvent is added below the numerical range, the purity of the terephthalic acid finally obtained may be reduced due to an emulsion layer between the aqueous phase and the organic phase, and if the organic solvent is added above the numerical range, the terephthalic acid may be separated due to the organic phase, thereby reducing the purity. In addition, if the amount of organic solvent for purification to be discarded increases, the economic feasibility decreases, and environmental pollution occurs.

The purifying of the terephthalate metal salt aqueous solution by treating the aqueous solution with the adsorbent is to remove trace impurities and organic solvents.

In the purifying of the terephthalate metal salt aqueous solution by treating the aqueous solution with the adsorbent, the adsorbent may be at least one selected from the group consisting of incineration ash, activated carbon, zeolite, silicate, calcium carbonate, calcium oxide, calcium hydroxide, magnesium carbonate, magnesium oxide, magnesium hydroxide, sodium carbonate, sodium bicarbonate, and alumina.

The precipitating of the solid terephthalic acid by treating the purified terephthalate metal salt aqueous solution with the acid solution is to obtain terephthalic acid by reacting the metal salt with acids. The acidic solution may use strong acids, and preferably, sulfuric acid may be used to precipitate terephthalic acid with high purity.

According to another embodiment of the present disclosure, the terephthalic acid produced by any one of methods for producing high-purity regenerated terephthalic acid using the polyester depolymerization may have a purity of 99.00% or higher. Preferably, the terephthalic acid may have a purity of 99.50% or higher. The regenerated terephthalic acid produced by any one of the producing methods is terephthalic acid having an extremely low impurity content.

A method for calculating the conversion rate of terephthalic acid by the producing method is as follows.

$$\text{Conversion rate (\%)} = \frac{(\text{mass (g) of added } \textit{PET} - \text{mass (g) of unreacted } \textit{PET})}{\text{mass (g) of added } \textit{PET}} \times 100$$

A method for calculating the yield of terephthalic acid by the producing method is as follows.

$$\text{Yield (\%)} = \frac{\text{mole of obtained } \textit{TPA}}{\text{mole of (mass (g) of added } \textit{PET} - \text{mass (g) of unreacted } \textit{PET})} \times 100$$

Hereinafter, the present disclosure will be described in more detail through Examples. These Examples are only to describe the present disclosure in more detail, and it will be apparent to those skilled in the art that the scope of the present disclosure is not limited by these Examples in accordance with the gist of the present disclosure.

**Examples and Comparative Examples**

**[Comparison of depolymerization reactions according to weight of polarity adjusting compound]**

Example 1

A solvent was prepared by adding 8.3 g of NaOH to a solvent mixed with 40 g of toluene and 60 g of ethanol in a 250 mL flask (S1).

10 g of finely crushed PET waste was added to the solvent prepared in step S1, and stirred at 60°C for 3 hours (S2).

A PET depolymerization product after the stirring was filtered to obtain a sludge cake, and 80 g of water was added to the filtered sludge cake to obtain an aqueous solution from which disodium terephthalate was eluted (S3).

20 g of hexane was treated to the aqueous solution to separate the phases, and only the aqueous layer was recovered (S4).

0.1 g of activated carbon was treated to the aqueous layer, stirred for 2 hours, and filtered to recover a purified aqueous layer (S5).

The recovered aqueous layer was treated with sulfuric acid to adjust the pH to 3 or lower, and a white solid was obtained, filtered, washed three times with 30 g of water, and dried to obtain white terephthalic acid (S6).

Example 2

Terephthalic acid was obtained in the same manner as in Example 1, except that 26 g of ethanol was applied in step S1.

Example 3

Terephthalic acid was obtained in the same manner as in Example 1, except that 40 g of ethanol was applied in step S1.

Example 4

Terephthalic acid was obtained in the same manner as in Example 1, except that 100 g of ethanol was applied in step S1.

Example 5

Terephthalic acid was obtained in the same manner as in Example 1, except that 4 g of ethanol was applied in step S1.

Example 6

Terephthalic acid was obtained in the same manner as in Example 1, except that 20 g of ethanol was applied in step S1.

Example 7

Terephthalic acid was obtained in the same manner as in Example 1, except that 200 g of ethanol was applied in step S1.

Example 8

Terephthalic acid was obtained in the same manner as in Example 1, except that 400 g of ethanol was applied in step S1.

Comparative Example 1

Terephthalic acid was obtained in the same manner as in Example 3, except that 40 g of anisole was applied instead of toluene in step S1.

Comparative Example 2

Terephthalic acid was obtained in the same manner as in Example 3, except that 40 g of MIBK was applied instead of toluene in step S1.

[Table 1]

| | PET (g) | Toluene (g) | EtOH (g) | NaOH (g) | Time (h) | Obtained TPA (g) | Yield (%) | Conversion rate (%) | Purity % (Acid value) |
|---|---|---|---|---|---|---|---|---|---|
| Ex. 1 | 10 | 40 | 60 | 8.3 | 3 | 8.62 | 99.71 | 100.00 | 99.67 |
| Ex. 2 | 10 | 40 | 26 | 8.3 | 3 | 8.59 | 99.36 | 100.00 | 99.88 |
| Ex. 3 | 10 | 40 | 40 | 8.3 | 3 | 8.62 | 99.71 | 100.00 | 99.86 |
| Ex. 4 | 10 | 40 | 100 | 8.3 | 3 | 8.56 | 99.02 | 100.00 | 99.56 |
| Ex. 5 | 10 | 40 | 4 | 8.3 | 3 | 8.11 | 95.65 | 98.06 | 99.63 |
| Ex. 6 | 10 | 40 | 20 | 8.3 | 3 | 8.14 | 94.26 | 99.87 | 99.74 |
| Ex. 7 | 10 | 40 | 200 | 8.3 | 3 | 7.14 | 96.38 | 85.69 | 99.66 |
| Ex. 8 | 10 | 40 | 400 | 8.3 | 3 | 6.17 | 94.87 | 75.26 | 99.54 |
| | PET (g) | Anisole (g) | EtOH (g) | NaOH (g) | Time (h) | Obtained TPA (g) | Yield (%) | Conversion rate (%) | Purity % (Acid value) |
| Com. Ex. 1 | 10 | 40 | 40 | 8.3 | 3 | 8.25 | 96.58 | 98.78 | 99.23 |
| | PET (g) | MIBK (g) | EtOH (g) | NaOH (g) | Time (h) | Obtained TPA(g) | Yield (%) | Conversion rate (%) | Purity % (Acid value) |
| Com. Ex. 2 | 10 | 40 | 40 | 8.3 | 3 | 8.12 | 95.65 | 98.20 | 99.14 |

[Comparison of depolymerization reactions according to weight of alkaline catalyst]

Example 9

Terephthalic acid was obtained in the same manner as in Example 1, except that 1 g of NaOH was applied in step S1.

Example 10

Terephthalic acid was obtained in the same manner as in Example 1, except that 5 g of NaOH was applied in step S1.

Example 11

Terephthalic acid was obtained in the same manner as in Example 1, except that 30 g of NaOH was applied in step S1.

Example 12

Terephthalic acid was obtained in the same manner as in Example 1, except that 50 g of NaOH was applied in step S1.

[Table 2]

| | PET (g) | Toluene (g) | EtOH (g) | NaOH (g) | Time( h) | Obtained TPA (g) | Yield (%) | Conversion rate (%) | Purity % (Acid value) |
|---|---|---|---|---|---|---|---|---|---|
| Ex. 1 | 10 | 40 | 60 | 8.3 | 3 | 8.62 | 99.71 | 100.00 | 99.67 |
| Ex. 9 | 10 | 40 | 60 | 1 | 3 | 1.01 | 95.35 | 12.21 | 99.61 |
| Ex. 10 | 10 | 40 | 60 | 5 | 3 | 3.69 | 94.22 | 45.32 | 99.65 |
| Ex. 11 | 10 | 40 | 60 | 30 | 3 | 8.58 | 99.21 | 100 | 99.54 |
| Ex. 12 | 10 | 40 | 60 | 50 | 3 | 8.61 | 99.61 | 100 | 99.89 |

[Comparison of depolymerization reactions according to weight of solvent]

Example 13

Terephthalic acid was obtained in the same manner as in Example 1, except that in step S1, 0.8 g of NaOH was added to a solvent mixed with 3.7 g of toluene and 5.5 g of ethanol to prepare a solvent.

Example 14

Terephthalic acid was obtained in the same manner as in Example 1, except that in step S1, 3.8 g of NaOH was added to a solvent mixed with 18.5 g of toluene and 27.7 g of ethanol to prepare a solvent.

Example 15

Terephthalic acid was obtained in the same manner as in Example 1, except that in step S1, 15 g of NaOH was added to a solvent mixed with 74 g of toluene and 111 g of ethanol to prepare a solvent.

Example 16

Terephthalic acid was obtained in the same manner as in Example 1, except that in step S1, 20 g of NaOH was added to a solvent mixed with 92 g of toluene and 138 g of ethanol to prepare a solvent.

[Table 3]

| | PET (g) | Toluene (g) | EtOH (g) | NaOH (g) | Time (h) | Obtained TPA (g) | Yield (%) | Conversion rate (%) | Purity % (Acid value) |
|---|---|---|---|---|---|---|---|---|---|
| Ex. 1 | 10 | 40 | 60 | 8.3 | 3 | 8.62 | 99.71 | 100.00 | 99.67 |
| Ex. 13 | 10 | 3.7 | 5.5 | 0.8 | 3 | 0.72 | 81.24 | 10.24 | 99.68 |
| Ex. 14 | 10 | 18.5 | 27.7 | 3.8 | 3 | 5.40 | 84.21 | 74.21 | 99.74 |
| Ex. 15 | 10 | 74 | 111 | 15 | 3 | 8.61 | 99.63 | 100 | 99.87 |
| Ex. 16 | 10 | 92 | 138 | 20 | 3 | 8.61 | 99.58 | 100 | 99.68 |

Example 17

A solvent was prepared by adding 8.3 g of NaOH to a solvent mixed with 40 g of toluene and 40 g of ethanol in a 250 mL flask (S1').

10 g of finely crushed PET waste was added to the solvent prepared in step S1, and stirred at 60°C for 3 hours (S2').

A PET depolymerization product after the stirring was filtered to obtain a sludge cake, and 80 g of water was added to the filtered sludge cake to obtain an aqueous solution from which disodium terephthalate was eluted. After obtaining the sludge cake, the solvent filtrate mixed with toluene and ethanol was stored for use in the next reaction (S3').

20 g of hexane was treated to the aqueous solution to separate the phases, and only the aqueous layer was recovered (S4').

0.1 g of activated carbon was treated to the aqueous layer, stirred for 2 hours, and filtered to recover a purified aqueous layer (S5').

The recovered aqueous layer was treated with sulfuric acid to adjust the pH to 3 or lower, and a white solid was obtained, filtered, washed three times with 30 g of water, and dried to obtain white terephthalic acid (S6').

The solvent was prepared by adding the solvent filtrate mixed with toluene and ethanol stored in step S3' and 4.5 g of NaOH to a 250 mL flask, and 10 g of finely crushed PET waste was treated with the solvent and stirred at 60°C for 3 hours. The subsequent process was repeated 10 times in total by repeating the processes of S3' to S6' (S7'). The conversion rate of toluene among them was graphed and shown in FIG. 1.

[Table 4]

| Times | PET (g) | Toluene (g) | EtOH (g) | NaOH (g) | Time (h) | Obtained TPA (g) | Yield (%) | Conversion rate (%) |
|---|---|---|---|---|---|---|---|---|
| 1 | 10 | 40 | 40 | 8.3 | 3 | 8.61 | 99.60 | 100.00 |
| 2 | 10 | | | 4.5 | 3 | 8.52 | 98.55 | 100.00 |
| 3 | 10 | | | 4.5 | 3 | 8.59 | 99.36 | 100.00 |
| 4 | 10 | | | 4.5 | 3 | 8.60 | 99.48 | 100.00 |
| 5 | 10 | | | 4.5 | 3 | 8.58 | 99.25 | 100.00 |
| 6 | 10 | Repeated use | | 4.5 | 3 | 8.55 | 98.90 | 100.00 |
| 7 | 10 | | | 4.5 | 3 | 8.59 | 99.57 | 99.79 |
| 8 | 10 | | | 4.5 | 3 | 8.55 | 99.22 | 99.68 |
| 9 | 10 | | | 4.5 | 3 | 8.50 | 98.91 | 99.41 |
| 10 | 10 | | | 4.5 | 3 | 8.53 | 99.63 | 99.04 |

Example 18

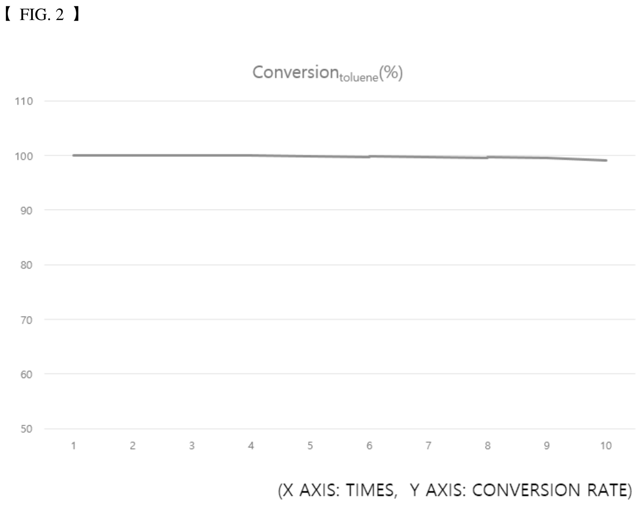

Terephthalic acid was obtained in the same manner as in Example 17, except that in step S1, 8.3 g of NaOH was added to a solvent mixed with 40 g of toluene and 28 g of ethanol to prepare a solvent. The conversion rate of toluene among them was graphed and shown in FIG. 2.

[Table 5]

| Times | PET (g) | Toluene (g) | EtOH (g) | NaOH (g) | Time (h) | Obtained TPA (g) | Yield (%) | Conversion rate (%) |
|---|---|---|---|---|---|---|---|---|
| 1 | 10 | 40 | 28 | 8.3 | 3 | 8.64 | 99.91 | 100.00 |
| 2 | 10 | | | 4.5 | 3 | 8.62 | 99.82 | 100.00 |
| 3 | 10 | | | 4.5 | 3 | 8.62 | 99.68 | 100.00 |
| 4 | 10 | | | 4.5 | 3 | 8.59 | 99.36 | 100.00 |
| 5 | 10 | | | 4.5 | 3 | 8.57 | 99.21 | 99.90 |
| 6 | 10 | Repeated use | | 4.5 | 3 | 8.60 | 99.58 | 99.87 |
| 7 | 10 | | | 4.5 | 3 | 8.59 | 99.68 | 99.71 |
| 8 | 10 | | | 4.5 | 3 | 8.60 | 99.85 | 99.66 |
| 9 | 10 | | | 4.5 | 3 | 8.57 | 99.63 | 99.51 |
| 10 | 10 | | | 4.5 | 3 | 8.56 | 99.92 | 99.12 |

Example 19

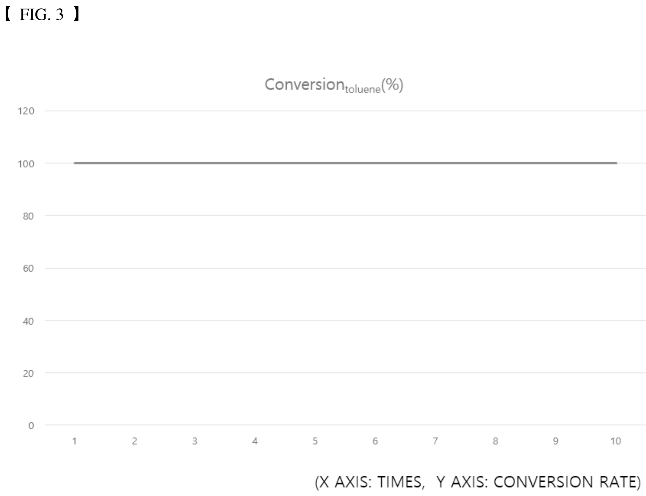

Terephthalic acid was obtained in the same manner as in Example 17, except that in step S1, 8.3 g of NaOH was added to a solvent mixed with 40 g of toluene and 52 g of ethanol to prepare a solvent. The conversion rate of toluene among them was graphed and shown in FIG. 3.

[Table 6]

| Times | PET (g) | Toluene (g) | EtOH (g) | NaOH (g) | Time (h) | Obtained TPA (g) | Yield (%) | Conversion rate (%) |
|---|---|---|---|---|---|---|---|---|
| 1 | 10 | 40 | 52 | 8.3 | 3 | 8.57 | 99.14 | 100.00 |
| 2 | 10 | | | 4.5 | 3 | 8.57 | 99.12 | 100.00 |
| 3 | 10 | | | 4.5 | 3 | 8.62 | 99.68 | 100.00 |
| 4 | 10 | | | 4.5 | 3 | 8.64 | 99.94 | 100.00 |
| 5 | 10 | | | 4.5 | 3 | 8.58 | 99.23 | 100.00 |
| 6 | 10 | Repeated use | | 4.5 | 3 | 8.63 | 99.81 | 100.00 |
| 7 | 10 | | | 4.5 | 3 | 8.60 | 99.45 | 100.00 |
| 8 | 10 | | | 4.5 | 3 | 8.61 | 99.63 | 100.00 |
| 9 | 10 | | | 4.5 | 3 | 8.58 | 99.21 | 100.00 |
| 10 | 10 | | | 4.5 | 3 | 8.59 | 99.36 | 100.00 |

Comparative Example 3

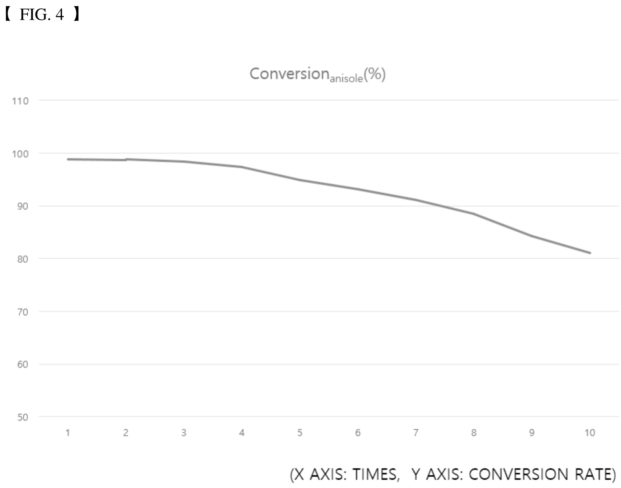

Terephthalic acid was obtained in the same manner as in Example 17, except that 40 g of anisole was applied instead of toluene in step S1' of Example 17. The conversion rate of anisole among them was graphed and shown in FIG. 4.

[Table 7]

| Times | PET (g) | Anisole (g) | EtOH (g) | NaOH (g) | Time (h) | Obtained TPA (g) | Yield (%) | Conversion rate (%) |
|---|---|---|---|---|---|---|---|---|
| 1 | 10 | 40 | 40 | 8.3 | 3 | 8.35 | 97.81 | 98.79 |

(continued)

| Times | PET (g) | Anisole (g) | EtOH (g) | NaOH (g) | Time (h) | Obtained TPA (g) | Yield (%) | Conversion rate (%) |
|---|---|---|---|---|---|---|---|---|
| 2 | 10 | | | 4.5 | 3 | 8.35 | 97.77 | 98.78 |
| 3 | 10 | | | 4.5 | 3 | 8.33 | 97.94 | 98.44 |
| 4 | 10 | | | 4.5 | 3 | 8.19 | 97.38 | 97.31 |
| 5 | 10 | | | 4.5 | 3 | 7.95 | 96.87 | 94.88 |
| 6 | 10 | Repeated use | | 4.5 | 3 | 7.85 | 97.53 | 93.09 |
| 7 | 10 | | | 4.5 | 3 | 7.73 | 98.07 | 91.16 |
| 8 | 10 | | | 4.5 | 3 | 7.47 | 97.75 | 88.44 |
| 9 | 10 | | | 4.5 | 3 | 7.06 | 96.92 | 84.31 |
| 10 | 10 | | | 4.5 | 3 | 6.80 | 97.06 | 81.04 |

Comparative Example 4

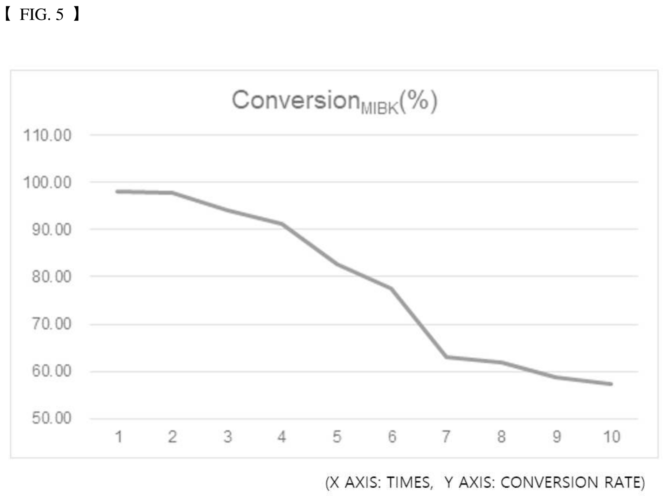

Terephthalic acid was obtained in the same manner as in Example 17, except that 40 g of MIBK was applied instead of toluene in step S1' of Example 17. The conversion rate of MIBK among them was graphed and shown in FIG. 5.

[Table 8]

| Times | PET (g) | MIBK (g) | EtOH (g) | NaOH (g) | Time (h) | Obtained TPA (g) | Yield (%) | Conversion rate (%) |
|---|---|---|---|---|---|---|---|---|
| 1 | 10 | 40 | 40 | 8.3 | 3 | 8.47 | 99.84 | 98.13 |
| 2 | 10 | | | 4.5 | 3 | 8.44 | 99.86 | 97.77 |
| 3 | 10 | | | 4.5 | 3 | 8.02 | 98.66 | 94.03 |
| 4 | 10 | | | 4.5 | 3 | 7.74 | 98.27 | 91.11 |
| 5 | 10 | | | 4.5 | 3 | 6.98 | 97.52 | 82.79 |
| 6 | 10 | Repeated use | | 4.5 | 3 | 6.64 | 98.92 | 77.65 |
| 7 | 10 | | | 4.5 | 3 | 5.21 | 95.57 | 63.06 |
| 8 | 10 | | | 4.5 | 3 | 5.12 | 95.74 | 61.86 |
| 9 | 10 | | | 4.5 | 3 | 5.06 | 99.63 | 58.75 |
| 10 | 10 | | | 4.5 | 3 | 4.89 | 98.70 | 57.31 |

**Claims**

1.  A method for producing high-purity regenerated terephthalic acid using polyester depolymerization, the method comprising:

preparing a solvent by mixing an alkaline catalyst with a basic hydrolysis solvent consisting of an alkylated aromatic compound and a polarity adjusting compound;
obtaining a polyester depolymerization product by treating waste polyester with the solvent;
preparing a sludge cake aqueous solution containing a terephthalate metal salt by filtering the polyester depolymerization product to obtain a sludge cake and adding purified water to the filtered sludge cake;
obtaining an aqueous solution containing a terephthalate metal salt by treating the sludge cake aqueous solution with an organic solvent;
purifying the terephthalate metal salt aqueous solution by treating the aqueous solution with an adsorbent; and
precipitating solid terephthalic acid by treating the purified terephthalate metal salt aqueous solution with an acidic solution.

2. The method of claim 1, wherein in the preparing of the solvent by mixing the alkaline catalyst with the basic hydrolysis solvent consisting of the alkylated aromatic compound and the polarity adjusting compound, the alkylated aromatic compound is at least one compound selected from the group consisting of toluene, xylene, trimethylbenzene, ethylbenzene, diethylbenzene, propylbenzene, dipropylbenzene, and butylbenzene.

3. The method of claim 1, wherein in the preparing of the solvent by mixing the alkaline catalyst with the basic hydrolysis solvent consisting of the alkylated aromatic compound and the polarity adjusting compound, the polarity adjusting compound is at least one compound selected from the group consisting of methanol, ethanol, propanol, butanol, pentanol, hexanol, heptanol, octanol, nonanol, decanol, undecanol, dodecanol, ethylene glycol, propylene glycol and butylene glycol.

4. The method of claim 1, wherein in the preparing of the solvent by mixing the alkaline catalyst with the basic hydrolysis solvent consisting of the alkylated aromatic compound and the polarity adjusting compound, the alkaline catalyst is at least one selected from the group consisting of potassium hydroxide, sodium hydroxide, lithium hydroxide, potassium oxide, sodium oxide, and lithium oxide.

5. The method of claim 1, wherein in the obtaining of the aqueous solution containing the terephthalate metal salt by treating the sludge cake aqueous solution with the organic solvent, the organic solvent is at least one selected from the group consisting of pentane, hexane, heptane, octane, nonane, decane, and undecane.

6. The method of claim 1, wherein in the purifying of the terephthalate metal salt aqueous solution by treating the aqueous solution with the adsorbent, the adsorbent is at least one selected from the group consisting of incineration ash, activated carbon, zeolite, silicate, calcium carbonate, calcium oxide, calcium hydroxide, magnesium carbonate, magnesium oxide, magnesium hydroxide, sodium carbonate, sodium bicarbonate, and alumina.

7. The method of claim 1, wherein in the preparing of the solvent by mixing the alkaline catalyst with the basic hydrolysis solvent consisting of the alkylated aromatic compound and the polarity adjusting compound, the weight ratio of the alkylated aromatic compound and the polarity adjusting compound is 1:0.1 to 10.

8. The method of claim 1, wherein in the preparing of the solvent by mixing the alkaline catalyst with the basic hydrolysis solvent consisting of the alkylated aromatic compound and the polarity adjusting compound, the weight ratio of the basic hydrolysis solvent and the alkaline catalyst is 1:0.01 to 0.5.

9. The method of claim 1, wherein in the obtaining of the polyester depolymerization product by treating the waste polyester with the solvent, the weight ratio of the waste polyester and the solvent is 1:1 to 50.

10. Regenerated terephthalic acid in which the terephthalic acid produced by the producing method of any one of claims 1 to 9 has the purity of 99.50% or higher.

【 FIG. 1 】

(X AXIS: TIMES,  Y AXIS: CONVERSION RATE)

【 FIG. 2 】

Conversion$_{toluene}$(%)

(X AXIS: TIMES, Y AXIS: CONVERSION RATE)

【 FIG. 3 】

Conversion$_{toluene}$(%)

(X AXIS: TIMES,  Y AXIS: CONVERSION RATE)

【 FIG. 4 】

Conversion$_{anisole}$(%)

(X AXIS: TIMES,  Y AXIS: CONVERSION RATE)

【 FIG. 5 】

(X AXIS: TIMES, Y AXIS: CONVERSION RATE)

# EP 4 653 416 A1

## INTERNATIONAL SEARCH REPORT

| | International application No. |
|---|---|
| | **PCT/KR2024/020791** |

### A. CLASSIFICATION OF SUBJECT MATTER

**C07C 51/02**(2006.01)i; **C07C 51/41**(2006.01)i; **C07C 51/09**(2006.01)i; **C07C 51/47**(2006.01)i; **C07C 63/26**(2006.01)i; **C07C 63/28**(2006.01)i; **C08J 11/24**(2006.01)i

According to International Patent Classification (IPC) or to both national classification and IPC

### B. FIELDS SEARCHED

Minimum documentation searched (classification system followed by classification symbols)

C07C 51/02(2006.01); C07C 51/09(2006.01); C07C 63/26(2006.01); C07C 67/02(2006.01); C07C 67/40(2006.01); C07C 69/82(2006.01); C08J 11/10(2006.01); C08J 11/18(2006.01); C08J 11/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폴리에스테르 해중합(polyester depolymerization), 재생 테레프탈산 (recycled terephthalic acid), 고순도(high purity), 알킬화 방향족 화합물(alkylated aromatic compound), 극성도(polarity index), 염기성 가수 분해(basic hydrolysis), 알칼리성 촉매(alkaline catalyst)

### C. DOCUMENTS CONSIDERED TO BE RELEVANT

| Category* | Citation of document, with indication, where appropriate, of the relevant passages | Relevant to claim No. |
|---|---|---|
| Y | KR 10-2024-0003512 A (KOREA RESEARCH INSTITUTE OF CHEMICAL TECHNOLOGY) 09 January 2024 (2024-01-09) See paragraphs [0044] and [0071]; and claims 1, 5, 7-8 and 10-12. | 1-6,10 |
| A | | 7-9 |
| Y | KR 10-2022-0037999 A (KOREA RESEARCH INSTITUTE OF CHEMICAL TECHNOLOGY) 25 March 2022 (2022-03-25) See claims 1 and 3-4. | 1-6,10 |
| Y | KR 10-2024-0033639 A (SK CHEMICALS CO., LTD.) 12 March 2024 (2024-03-12) See paragraphs [0048]-[0049]; and claims 1 and 10. | 1-6,10 |
| A | KR 10-2452867 B1 (KOREA RESEARCH INSTITUTE OF CHEMICAL TECHNOLOGY) 11 October 2022 (2022-10-11) See entire document. | 1-10 |

☑ Further documents are listed in the continuation of Box C.     ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **28 March 2025** | **28 March 2025** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/020791**

**C. DOCUMENTS CONSIDERED TO BE RELEVANT**

| Category* | Citation of document, with indication, where appropriate, of the relevant passages | Relevant to claim No. |
|---|---|---|
| A | WO 2024-005446 A1 (KOREA RESEARCH INSTITUTE OF CHEMICAL TECHNOLOGY) 04 January 2024 (2024-01-04)<br>See entire document. | 1-10 |

## INTERNATIONAL SEARCH REPORT
### Information on patent family members

International application No.

**PCT/KR2024/020791**

| Patent document cited in search report | | | Publication date (day/month/year) | Patent family member(s) | | | Publication date (day/month/year) |
|---|---|---|---|---|---|---|---|
| KR | 10-2024-0003512 | A | 09 January 2024 | KR | 10-2774087 | B1 | 04 March 2025 |
| | | | | WO | 2024-005445 | A1 | 04 January 2024 |
| KR | 10-2022-0037999 | A | 25 March 2022 | CN | 116635462 | A | 22 August 2023 |
| | | | | EP | 4183823 | A1 | 24 May 2023 |
| | | | | EP | 4183823 | A4 | 25 September 2024 |
| | | | | JP | 2023-541162 | A | 28 September 2023 |
| | | | | JP | 7473741 | B2 | 23 April 2024 |
| | | | | KR | 10-2462599 | B1 | 03 November 2022 |
| | | | | US | 2024-0417527 | A1 | 19 December 2024 |
| | | | | WO | 2022-060153 | A1 | 24 March 2022 |
| KR | 10-2024-0033639 | A | 12 March 2024 | CN | 118679140 | A | 20 September 2024 |
| | | | | EP | 4458800 | A1 | 06 November 2024 |
| | | | | KR | 10-2648916 | B1 | 19 March 2024 |
| | | | | WO | 2024-053928 | A1 | 14 March 2024 |
| KR | 10-2452867 | B1 | 11 October 2022 | CN | 117203182 | A | 08 December 2023 |
| | | | | EP | 4299556 | A1 | 03 January 2024 |
| | | | | JP | 2024-514136 | A | 28 March 2024 |
| | | | | KR | 10-2452871 | B1 | 11 October 2022 |
| | | | | US | 2024-0166590 | A1 | 23 May 2024 |
| | | | | WO | 2022-220543 | A1 | 20 October 2022 |
| WO | 2024-005446 | A1 | 04 January 2024 | KR | 10-2024-0003511 | A | 09 January 2024 |
| | | | | KR | 10-2735393 | B1 | 28 November 2024 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 102462599